# EUROPÄISCHE PATENTSCHRIFT

(11) **EP 1 532 436 B1**
(45) Veröffentlichungstag und Bekanntmachung des Hinweises auf die Patenterteilung: **04.03.2009**
(21) Anmeldenummer: 03797199.1
(22) Anmeldetag: 11.07.2003
(51) Int. Cl.: G01N 33/18, C08J 3/075

(54) **IONENSTÄRKE-SENSOR**
ION STRENGTH SENSOR
CAPTEUR DE LA FORCE IONIQUE

(30) Priorität: 21.08.2002 DE 10239204
(43) Veröffentlichungstag der Anmeldung: 25.05.2005
(73) Patentinhaber: Zahn, Frank, 76199 Karlsruhe (DE)
(72) Erfinder: ZAHN, Frank, 76199 Karlsruhe (DE); BOSSMANN, Stefan, H., Manhattan, Kansas 66503 (US)
(74) Vertreter: Walkenhorst, Andreas
(86) Internationale Anmeldenummer: PCT/EP2003/007507
(87) Internationale Veröffentlichungsnummer: WO 2004/027414

(56) Entgegenhaltungen:
- US-B1- 6 180 288
- JACKSON DK, ET AL.: "A Sensor for Measuring Gel Phase-Transition Temperature, with Potential as a Metal Ion Detector" J. INTELL. MATER. SYST. STRUCT., Bd. 8, Nr. 2, Seiten 184-190, XP009020816
- HOLTZ J H ET AL: "INTELLIGENT POLYMERIZED CRYSTALLINE COLLOIDAL ARRAYS: NOVEL CHEMICAL SENSOR MATERIALS" ANALYTICAL CHEMISTRY, AMERICAN CHEMICAL SOCIETY. COLUMBUS, US, Bd. 70, Nr. 4, 15. Februar 1998 (1998-02-15), Seiten 780-791, XP000738808 ISSN: 0003-2700

## Beschreibung

Die Erfindung bezieht sich auf einen Sensor zur Ermittlung der Ionenstärke zwei- und/oder höherwertiger Kationen in einem Medium mit einer mit dem Medium in Kontakt bringbaren Sensoreinheit. Sie betrifft weiter ein Verfahren zur Ermittlung der lonenstärke zwei- und/oder höherwertiger Kationen in einem Medium.

Die Ionenstärke ist eine Kenngröße von Lösungen. Sie bestimmt die biophysika-lischen Eigenschaften von Molekülen, wie zum Beispiel deren thermodynamische Stabilität, Löslichkeit und katalytische Aktivität. Eine hohe Ionenstärke schwächt die elektrostatischen Wechselwirkungen von Molekülen in Lösung und verstärkt deren hydrophobe Wechselwirkungen. Die Ionenstärke I einer Lösung berechnet sich in erster Näherung aus den Ladungen und Konzentrationen aller vorhandenen Ionen nach folgender Formel: I = 0,5Σnᵢ²·cᵢ. Dabei ist n die Ladung des betreffenden Ions und c dessen Konzentration.

Die Konzentration der zweiwertigen Erdalkaliionen, insbesondere der Calcium- und Magnesiumionen, in Wasser ist ein wesentliches Maß für die sogenannte Wasserhärte. Die Wasserhärte teilt sich auf in die sogenannte Carbonathärte oder temporäre Härte für die Hydrogencarbonate des Calcium(II)- und/oder Magnesium(II)-lons und in die sogenannte Nichtcarbonathärte oder permanente Härte für die entsprechenden Sulfate, Nitrate und Chloride. Die Härte des Wassers wird in Härtegraden angegeben. Ein "deutscher Grad" ("Wasserhärte", °d, °dH, D.G.) ist beispielsweise gemäß Jander-Blasius, Einführung in das anorganisch-chemische Praktikum, S. Hirzel Verlag, Stuttgart, 1984, definiert als 7.13 mg Ca (24.32 mg CaSO₄, 1,779 x 10⁻⁴ mol/L) oder 4.33 mg Mg (21.44 mg MgSO₄, 1,779 x 10⁻⁴ mol/L) in einem Liter Wasser. Im Allgemeinen wird die folgende Unterteilung der Wasserhärten in drei "Härtebereiche" vorgenommen:
I. 0 < D.G. < 4 (sehr) weiches Wasser
II. 4 < D.G. < 18 mittelhartes Wasser
III. 18 < D.G. hartes Wasser

Zu hartes Wasser kann einerseits zu Ablagerungen, insbesondere zur Bildung von sogenanntem Kesselstein an von Wasser durchströmten Komponenten, wie beispielsweise an Wasch- oder Kaffeemaschinen und Wasserrohren oder-kesseln im Haushalt oder in der Industrie führen. Diese Ablagerungen können zur Schädigung der Installationen und zum höheren Energieverbrauch aufgrund verschlechterter Wirkungsgrade führen. Andererseits bedingt beispielsweise die Ausfällung fettsaurer Calcium- und/oder Magnesiumsalze eine stark reduzierte Wasch- und Reinigungswirkung der auf Fettsäurebasis hergestellten Seifen und kann so beim Waschen zu einem höheren Verbrauch an Waschmitteln und zum Brüchigwerden des Gewebes führen.

Um die durch die Härte des Wassers verursachten Schäden und Beeinträchtigungen zu verhindern, kommen insbesondere aus Gründen der Werterhaltung von Installationen technischer Anwendungen sowie aus Umweltschutzgründen in zunehmendem Maße sowohl im Haushaltsbereich als auch im industriellen Bereich vielfältige Wasserenthärtungsanlagen zum Einsatz.

Für diese Enthärtungsanlagen gibt es in der Bundesrepublik Deutschland ein Prüfzeichen des deutsche Vereins des Gas- und Wasserfaches e.V. (DVGW), das ihre Sicherheit, Hygiene und Wirksamkeit garantiert. Während die sogenannte Carbonathärte oder temporäre Härte bereits durch das Kochen der Lösung reduziert werden kann, da z.B. nach der Gleichung Ca(HCO₃)₂ → CO₂ + H₂O + CaCO₃ Calciumhydrogencarbonat zu Calciumcarbonat umgewandelt und auf-grund dessen Schwerlöslichkeit ausgefällt wird, kann die durch die sogenannte Nichtcarbonathärte verursachte permanente Härte nicht durch Kochen des Wassers beseitigt werden. Die Enthärtung von Wasser für technische Zwecke erfolgt daher üblicherweise entweder durch Destillation des Wassers, durch chemische Ausfällung der störenden Ionen, durch Komplexbildung mit Polyphosphaten oder bevorzugt durch Entfernung der Ionen mittels anorganischer oder organischer Ionenaustauscher. Die technisch üblichen Mischbettaustauscher tauschen Erdalkalimetallkationen und höhere Kationen gegen Alkalimetallkationen aus. Alle Anionen werden gegen Chlorid ausgetauscht.

Für einen geeigneten Einsatz solcher Enthärtungsanlagen ist die Wasserhärte, also insbesondere die Ionenstärke der zweiwertigen Erdalkaliionen Ca²⁺ und Mg²⁺, regelmäßig zu bestimmen.

Zur Bestimmung der Wasserhärte wird als gängiges Verfahren die komplexo-metrische Titration gegen Ethyiendiamintetraessigsäure (EDTA) angewendet. Dabei wird die zu bestimmende Lösung mit einem Indikator versetzt, der mit dem Zielmetallion einen Komplex bildet und dabei sein Absorptionsverhalten verändert. Ein gängiges System als Indikator ist beispielsweise Eriochromschwarz T, das in Lösung eine blaue Färbung erzeugt, die nach rot umschlägt, wenn es mit Magnesiumionen einen Komplex bildet. Bei der Titration werden durch die Zugabe von EDTA die freien zwei- und höherwertigen Metallionen komplexiert bis schließlich auch dem Indikator-Metallionen-Komplex die Metallionen entzogen werden. Dabei kommt es zu einem Farbumschlag, der den Äquivalenzpunkt kennzeichnet. Aus dem Verbrauch an EDTA kann dann die Konzentration der zwei- und/oder höherwertigen Metallionen berechnet werden. Die komplexo-metrische Bestimmung von Mg²⁺ oder Ca²⁺ muss allerdings unter strenger pH-Kontrolle vorgenommen werden (pH 10,2 - 10,5). Außerdem reagiert Eriochrom-schwarz T schlechter mit Ca²⁺ als mit Mg²⁺. Dies führt zu einem unscharfen Umschlagsverhalten bei der Bestimmung der Wasserhärte, wenn Mg²⁺ und Ca²⁺ nebeneinander vorliegen.

Die Carbonathärte, genau die Pufferkapazität des Carbonats in der Lösung, ist prinzipiell auch durch Titration mit verdünnter Salzsäure zu ermitteln. Allerdings ist lediglich die Pufferkapazität zugänglich unbeachtlich der tatsächlichen Calcium- oder Magnesiumkonzentrationen. Ebenso würden andere Puffersubstanzen, den so ermittelten Wert der Carbonathärte verfälschen.

Zur Bestimmung der Ionenstärke von ausgewählten Metallionen kommt weiterhin eine potentiometrische Titration in Betracht. So wird z.B. für die Simultanbestimmung von Calcium und Magnesium im Wasser oder die Bestimmung der Gesamthärte mit EDTA eine Calcium-Elektrode verwendet. Diese zeigt aber häufig eine ungenügende lonenselektivität. Aufwändige Messkettensysteme, wie beispielsweise ionenselektive lonophore, gestatten auch die ionenselektive Konzentrationsbestimmung, rechtfertigen ihren Aufwand aber eher in der Spuren- und Präzisionsanalytik.

Diesen regelmäßig anzuwendenden Verfahren ist gemeinsam, dass sie von Verbrauchsstoffen abhängig sind, die richtig dosiert werden müssen. Ferner beinhalten die Titrationsverfahren aufwändige Einzelschritte wie die Bereitstellung von Maßlösungen. Weiterhin ist daher üblicherweise eine Probenentnahme erforderlich, die bereits an sich aufwändig ist und somit unerwünscht sein kann. Diese Verfahren sind also naturgemäß diskontinuierlich. Sie eignen sich somit schlecht für eine Automatisierung, was in der Regel mit einem recht hohen Personalbedarf verbunden ist, und führen üblicherweise zu einer zeitlich verzögerten Bestimmung der Ionenstärke.

Zur Minimierung möglicher Ausfallzeiten und Kosten durch die Wartung der von Wasser durchströmten Komponenten, die Reparatur oder den Ersatz der durch die Wasserhärte geschädigten Installationsteile kommt aber gerade der aktuellen und zeitnahen Ermittlung der Ionenstärke im zu bestimmenden Medium eine besondere Bedeutung zu. Für den rechtzeitigen Einsatz von geeigneten Enthärtungsanlagen ist also eine ständige, unkomplizierte Überwachung und zeitgleiche Bestimmung der Ionenstärke der für die Wasserhärte verantwortlichen Metallkationen wünschenswert.

Der Erfindung liegt daher die Aufgabe zugrunde, einen Sensor bereitzustellen, der auch bei einem gering gehaltenen Aufwand eine direkte und kontinuierliche Ermittlung der Ionenstärke zwei- und/oder höherwertiger Kationen erlaubt. Des Weiteren soll ein zur unaufwändigen, direkten und kontinuierlichen Ermittlung der Ionenstärke zwei- und/oder höherwertiger Kationen besonders geeignetes Verfahren angegeben werden. Bezüglich des Sensors wird diese Aufgabe erfindungsgemäß gelöst, indem er zur Ermittlung der Ionenstärke zwei- und/oder höherwertiger Kationen in einem Medium eine mit dem Medium in Kontakt bringbare Sensoreinheit aufweist, die als Sensormaterial ein Copolymer aufweist, das N-isopropyl-acrylamid (NIPAM) und ein Komplexbildner-Monomer (KMX) mit mindestens einer Carbonsäure-Gruppe umfasst, und das der allgemeinen Formel NIPAMₘ KMXₙ mit m ≥ 1 und n ≥ 1 genügt, und wobei das Copolymer in einem von einer Membran umgebenen Messraum bei einem pH-Wert von 6-8 vorgehalten ist.

Die Erfindung geht dabei von der Überlegung aus, dass der Sensor unter konsequenter Vermeidung von separaten Probenahmen für eine Anwendung am eigentlich zu messenden Medienstrom mit den zwei- und/oder höherwertigen Kationen, deren lonenstärke zu bestimmen ist, geeignet sein sollte. Daher weist der Sensor eine mit diesem Medium in Kontakt bringbare Sensoreinheit mit einem Sensormaterial auf. Die Sensoreinheit sollte ein Sensormaterial aufweisen, das von außen messbare Eigenschaftsänderungen abhängig von der Ionenstärke zwei- und/oder höherwertiger Kationen zeigt. Eine solche von außen messbare Eigenschaftsänderung ist über ein Sensormaterial realisierbar, dessen optische Eigenschaften sich abhängig von der lonenstärke ändern. Dazu weist die Sensoreinheit als Sensormaterial ein Copolymer auf, das abhängig von der Ionenstärke allein zwei- und/oder höherwertiger Kationen eine optisch detektierbare Phasenseparation infolge Koagulation zeigt. Dafür eignet sich ein Copolymer, das N-isopropyl-acrylamid (NIPAM) und ein Komplexbildner-Monomer (KMX) mit mindestens einer Carbonsäure-Gruppe umfasst, und das der allgemeinen Formel NIPAMₘ KMXₙ mit m ≥ 1 und n ≥ 1 genügt. Damit kann sowohl ein binäres Copolymer gemeint sein, das heißt ein Copolymer, das NIPAM und ausschließlich untereinander gleichartige Komplexbildner-Monomere umfasst, als auch ein Copolymer, das NIPAM und verschiedene Komplexbildner-Monomere umfasst.

Ein derartig aufgebautes Coploymer liegt nämlich in Form von individuellen Polymersträngen vor, bei denen jeweils der durch NIPAM bereitgestellte Strang mittels des Komplexbildner-Monomers durch eine Carbonsäure-Gruppe ergänzt ist. In geeigneter Umgebung liegt diese deprotoniert und somit als Carboxylat-Gruppe vor. Bei einer Zuführung von Kationen bildet diese Carboxylatgruppe Komplexe, wobei aufgrund der damit verbundenen Raumladungsverschiebung im Polymerstrang eine mikroskopische Zusammenballung auftritt, die bei geeigneter Einstellung der Umgebungsparameter in einer Koagulation oder Clusterbildung auch größerer Moleküleinheiten münden kann. Dies kann zu einer Eintrübung der makroskopischen Substanz führen, die mit optischen Mitteln detektierbar ist. Aufgrund dieses Mechanismus ist somit ein Rückschluß von den optischen Eigenschaften des Materials auf die quantitative Anwesenheit von Kationen möglich.

Das Sensormaterial sollte im Übrigen zur Vermeidung von störenden Einflüssen durch für die zu ermittelnde Ionenstärke nicht relevante Ionen so ausgestaltet sein, dass es mit den zu ermittelnden zweiwertigen Erdalkalimetallkationen, wie und Ca²⁺ und Mg²⁺, und/oder höherwertigen Metallkationen bedeutend stärker in Wechselwirkung tritt als mit einwertigen Kationen, wie den Alkalimetallkationen Na⁺ und K⁺, die im Wasser in bedeutend höherer Konzentration vorliegen. Gerade dieses Kriterium wird vom Copolymer der genannten Form in besonderem Maße erfüllt.

Als Maß für die für die genannte Wechselwirkung charakteristische Bindungsstärke gilt die freie Enthalpie ΔG. Unter Verwendung des Tetranatriumsalzes der Ethlyendiamintetraessigsäure (Na₄EDTA) als komplexometrisches Reagenz ergibt sich für die Bindungsstärke nach Jander-Blasius eine freie Enthalpie von ΔG = - 61,451 KJ/mol für Ca²⁺ und ΔG = - 49,907 KJ/mol für Mg²⁺ aus der Gleichung ΔG = - R T InK mit R als Gaskonstante [8,314 JK⁻¹mol⁻¹], T als Temperatur [K] und K als thermodynamische Gleichgewichtskonstante K = [M EDTA ⁻⁴⁺ⁿ ]/ [Na₄EDTA] [M^{n+]}. Diese Bindung wird dabei von den einwertigen Alkalimetallionen aufgrund deren sehr geringer Bindungsenthalpie, nämlich für Na⁺ lediglich ΔG = - 9,53 KJ/mol und für K⁺ ΔG = - 6,32 KJ/mol, nicht gestört. Höherwertige Kationen als zweiwertige Kationen führen generell zu höheren Bindungsenthalpien als für Ca²⁺ und Mg²⁺. Sie könnten daher grundsätzlich die Bestimmung der Erdalkalimetallkationen stören. Da jedoch auch diese höheren Kationen für Ablagerungen wie Kesselstein beim Erhitzen verantwortlich sind, sollten auch sie entfernt werden. Es ist daher besonders von Vorteil, wenn das Sensormaterial auch für sie ein guter Rezeptor oder Komplexbildner ist. Als selektiver Rezeptor oder Komplexbildner für zwei- und/oder höherwertige Kationen ist also als Sensormaterial ein Copolymer geeignet, das mindestens eine Carbonsäure-Gruppe aufweist oder insbesondere mit EDTA strukturell verwandt ist.

Für eine besonders günstige Nachweisbarkeit der zu bestimmenden Ionenstärke zwei- und/oder höherwertiger Kationen ist die Massenkonzentration des N-isopropyl-acrylamids (NIPAMₘ) im Copolymer vorzugsweise größer 50% gewählt.

Dabei weist das Komplexbildner-Monomer (KMX) vorteilhafterweise mit zwei- und/oder höherwertigen Kationen eine freie Bindungsenthalpie kleiner als ΔG = - 35 KJ/mol auf.

In weiterer vorteilhafter Ausgestaltung ist als Komplexbildner-Monomer (KMX) Acrylsäure, {[4-Acryloyl-amino-2-(bis-carboxy-methyl-amino)-phenyl]-carboxy-methyl-amino}-essigsäure, {[4-Acryloyl-amino-2-(bis-carboxy-methyl-amino)-cyclohexyl]-carboxymethyl-amino}-essigsäure, ({2-[Acryloyl-(2-dicarboxymethylamino-ethyl)-amino]-ethyl}-carboxymethyl-amino)-essigsäure, [({Acryloyl-[(dicarboxymethyl-amino)-methyl]-amino}-methyl)-carboxymethyl-amino]-essigsäure, Methacrylsäure, organische Acrylate, organische Methacrylate, Zimtsäure oder Derivate der Zimtsäure eingesetzt. Dabei sind alle vorgenannten Komplexbildner-Monomere (KMX) vorzugsweise mit einer Massenkonzentration von 0,01-2% verwendet, mit Ausnahme von Methacrylsäure, das vorzugsweise mit einer Massenkonzentration von 1-2% eingesetzt ist. Die Synthese eines binären Copolymers oder eines verschiedene Komplexbildner-Monomere umfassenden Copolymers erfolgt dabei insbesondere über radikalische, kationische oder anionische Polymerisation.

Die für die Ionenstärke charakteristische Phasenseparation des Copolymers infolge Koagulation ist umso leichter nachzuweisen oder auswertbar, umso größer die beteiligten Moleküle sind. Daher weist das Copolymer vorzugsweise eine mittlere Molekularmasse im Bereich zwischen 40.000 und 200.000 auf.

Allgemein sind in der technischen Anwendung drei Härtebereiche zu unterscheiden:

| | | |
|---|---|---|
| I. | 0 < D.G. < 10⁻³ | (z.B. Kesselspeisewasser) |
| II. | 10⁻³ < D.G. < 1 | (z.B. entionisiertes Wasser für viele technische Anwendungen) |
| III. | D.G. > 1 | (z.B. Wasser für die Waschmaschine) |

Für solch einen vorgegebenen Messbereich ist das Copolymer vorzugsweise jeweils hinsichtlich seines Gehalts an Carbonsäure-Gruppen und/oder deren Art angepasst, was insbesondere durch eine geeignete Auswahl des Komplexbildner-Monomers erfolgen kann.

Dabei sind für den Messbereich 0 < D.G. < 10 ⁻³ prinzipiell alle Ca²⁺- und Mg²⁺- Komplexbildner verwendbar, die zweckmäßigerweise chemisch mit N-isopropyl-acrylamid verknüpft werden können und deren freie Bindungsenthalpie kleiner als ΔG = - 50 KJ/mol ist. Dafür eignet sich vorzugsweise als Komplexbildner-Monomer {[4-Acryloylamino-2-(bis-carboxy-methyl-amino)-phenyl]-carboxy-methyl-amino}-essigsäure, das eine theoretische freie Bindungsenthalpie bei Ca²⁺ von ΔGₜₕₑₒ = - 60,1 KJ/mol und bei Mg²⁺ von ΔGₜₕₑₒ = - 51,2 KJ/mol aufweist, oder das Komplexbildner-Monomer {[4-Acryloyl-amino-2-(bis-carboxy-methyl-amino)-cyclohexyl]-carboxy-methyl-amino}-essigsäure, das eine theoretische freie Bindungsenthalpie bei Ca²⁺ von ΔGₜₕₑₒ = - 62,7 KJ/mol und bei Mg²⁺ von ΔGₜₕₑₒ = - 52,9 KJ/mol aufweist.

Für den Messbereich 10 ⁻³ < D.G. < 1 sind prinzipiell alle Ca²⁺ und Mg²⁺ - Rezeptoren verwendbar, die zweckmäßigerweise chemisch mit N-isopropyl-acrylamid verknüpft werden können und deren freie Bindungsenthalpie zwischen ΔG = - 50 KJ/mol und - 35 KJ/mol liegt. Dafür ist vorzugsweise als Komplexbildner-Monomer ({2-[Acryloyl-(2-dicarboxymethylamino-ethyl)-amino]-ethyl}-carboxymethyl-amino)-essigsäure, das eine theoretische freie Bindungsenthapie bei Ca²⁺ von ΔGₜₕₑₒ = - 43,0 KJ/mol und bei Mg²⁺ von ΔGₜₕₑₒ = 40,7 KJ/mol aufweist, oder [({Acryloyl-[(dicarboxymethyl-amino)-methyl]-amino}-methyl)-carboxymethyl-amino]-essigsäure, das eine theoretische freie Bindungsenthalpie bei Ca²⁺ von ΔGₜₕₑₒ = - 44,9 KJ/mol und bei Mg²⁺ von ΔGₜₕₑₒ = - 41,0 KJ/mol aufweist, eingesetzt.

Für den Messbereich von D.G.> 1 ist vorzugsweise als Komplexbildner-Monomer Acrylsäure, Methacrylsäure, organisches Acrylat, organisches Methacrylat, Zimtsäure oder ein Derivat der Zimtsäure vorgesehen, da die Wechselwirkung ihrer Carbonsäure-Gruppe ausreicht, um eine Abhängigkeit der Phasenseparation infolge Koagulation von der Ionenstärke zwei- und/oder höherwertiger Kationen, beispielsweise von der Wasserhärte, zu gewährleisten. Ferner sind diese Stoffe im Allgemeinen für die industrielle Einsetzbarkeit leicht verfügbar.

Für die industrielle Einsetzbarkeit des Sensors ist des Weiteren dessen sogenannte Standzeit, also die Zeit seines durchgehenden und ununterbrochenen Einsatzes, bedeutsam. Diese Standzeit könnte durch Löslichkeitsverluste des als Sensormaterial verwendeten Copolymers begrenzt werden. Wie sich herausgestellt hat, ist für die Löslichkeit die Molekularmasse des Copolymers von besonderer Bedeutung. Bei einer zu niedrigen Molekularmasse kommt es bereits bei Standzeiten von einigen Stunden oder Tagen zu Löslichkeitsverlusten des verwendeten Copolymers, da dieses durch die Poren einer verwendeten Membran in das zu messende Medium diffundiert. Um die Löslichkeitsverluste des Copolymers so weit zu verringern, dass Standzeiten bis hin zu einem Jahr erreichbar sind, sind die individuellen Polymerstränge des Copolymers vorteilhafterweise zur Bildung eines Makromoleküls vernetzt, wobei das durch die Vernetzung des Copolymers gebildete Makromolekül zweckmäßigerweise eine Molekularmasse größer 2.000.000 aufweist.

Zur ausreichenden chemischen Langzeitstabilität erfolgt die Vernetzung der individuellen Polymerstränge des Copolymers insbesondere über eine C-C-Bindung, zweckmäßigerweise über Diene beliebiger Struktur, vorzugsweise über Divinylbenzol oder substituierte Butadiene als Vernetzer. Dabei können die Vernetzer des Copolymers während der Synthese des Copolymers statistisch in das Copolymer eingebaut werden. Allerdings kann bei einer Vernetzung der Copolymere während der Synthese nachfolgend die Koagulation individueller Polymerstränge durch Sekundäreffekte der Vernetzung behindert werden. In der Folge kann häufig in Abhängigkeit des Vernetzungsgrades nur noch ein An- und Abschwellen des durch die Vernetzung entstandenen Hydrogels beobachtet werden. Dies kann zu einem signifikanten Verlust an Empfindlichkeit bei der optischen Messung der Ionenstärke zwei- und/oder höherwertiger Kationen, beispielsweise der Wasserhärte, führen.

Daher erfolgt die Vernetzung der individuellen Polymerstränge des Copolymers für eine ausreichende chemische Langzeitstabilität alternativ vorteilhafterweise unter Knüpfung einer Carbonsäureamidbindung über Amin-terminierte Polyoxyethylen-Brücken, Amin-terminierte Polyoxyethylen-Brücken-substituierte Aromaten, Amin-terminierte Polyimine, Amin-terminierte aliphatische Kohlenwasserstoffe, Amin-terminierte aliphatisch-aromatische Kohlenwasserstoffe mit beliebigem Substitutionsmuster oder eine Kombination der vorgenannten Strukturelemente in einem zweiten Schritt nach der Synthese des eigentlichen Copolymers.

Die Sensoreinheit weist erfindungsgemäß als aktives Sensormaterial eine wässrige Lösung des Copolymers auf. Diese ist im Inneren eines Messraumes vorgehalten, der zweckmäßigerweise von einer Membran umgeben ist. Für einen ausreichenden Kontakt zwischen dem Sensormaterial und dem zu vermessenden Medium ist die Membran vorzugsweise für zwei- und/oder höherwertige Kationen permeabel. Das heißt, die zwei- und/oder höherwertigen Kationen können von außen durch die Membran in den Messraum diffundieren.

Eine besonders günstige und zudem berührungslose Auswertung der für die Ionenstärke charakteristischen Koagulation des Sensormaterials in wässriger Lösung ist erreichbar, indem in besonders vorteilhafter Ausgestaltung eine Erfassung der optischen Eigenschaften des Sensormaterials in wässriger Lösung vorgesehen ist, zumal sich die Koagulation der einzelnen Polymerstränge in einer Trübung der Lösung niederschlägt. Zur Auswertung der optischen Eigenschaften des Copolymers in Abhängigkeit von der Ionenstärke zwei- und/oder höherwertiger Kationen sind der mit dem Copolymer als Sensormaterial versehenen Sensoreinheit eine Lichtquelle und ein Lichtdetektor zugeordnet.

Als Lichtquelle kommt eine Leuchtdiode oder eine beliebige Lampe in Betracht. Um eine monochromatische Bestrahlung der mit dem Copolymer als Sensormaterial versehenen Sensoreinheit zu gewährleisten, ist eine als Lichtquelle gewählte Lampe beispielsweise mit entsprechenden Filtern versehen oder als Lichtquelle ein Laser eingesetzt. Eine polychromatische Bestrahlung ist jedoch ebenfalls möglich. Für einen technisch relevanten Einsatzbereich mit einem geringen Kosten- und Herstellungsaufwand ist die Lichtquelle vorteilhafterweise für eine Bestrahlung mit einer Wellenlänge oder einem Wellenlängenintervall im Bereich von 300 nm bis 500 nm eingestellt.

Wie sich herausgestellt hat, ist der pH-Wert für die optisch detektierbare Phasenseparation des Copolymers von besonderer Bedeutung. Dafür ist das Copolymer vorzugsweise von einem Polymer, beispielsweise von Polyethylenimid, umgeben, das den pH-Wert der wässrigen Lösung des Copolymers puffert. Die Abhängigkeit vom pH-Wert ist des Weiteren in besonders vorteilhafter Ausgestaltung mittels eines Medienüberwachungssystems, das der mit dem Copolymer als Sensormaterial versehenen Sensoreinheit zugeordnet ist, berücksichtigt. Das Medienüberwachungssystem umfasst dabei vorzugsweise einen pH-Sensor und eine Zuspeiseeinheit für ein Zuspeisemittel, das eine Abweichung von dem gewünschten pH-Wert kompensieren kann. Um den intrinsischen pH-Wert des Copolymers, der in wässriger Lösung je nach der Konzentration des jeweiligen Copolymer (1 - 50 g/L) bei einem pH-Wert zwischen 5 und 3 liegt, auf sich als günstig herausgestellte pH-Werte von pH = 6, 7 oder 8 zu erhöhen, ist als Zuspeisemittel vorzugsweise Natronlauge eingesetzt.

Ferner hat sich eine Abhängigkeit der optisch detektierbaren Phasenseparation des Copolymers von der Temperatur herausgestellt. Um eine für die Ermittlung der lonenstärke zwei- und/oder höherwertiger Kationen geeignete Temperatur zu erzielen, die höher ist als Raumtemperatur, ist die mit dem Copolymer als Sensormaterial versehene Sensoreinheit vorzugsweise mit einem Temperatur-Sensor und einem Heizelement versehen, das vorzugsweise auf eine konstante Temperatur von 40, 45, 50, 55, 60, 65 oder 70 °C heizt.

Bezüglich des Verfahrens zur Ermittlung der Ionenstärke zwei- und/oder höherwertiger Kationen in einem Medium wird die genannte Aufgabe gelöst, indemjn Abhängigkeit von der Ionenstärke ein optischer Transmissionsgrad einer wässrigen Lösung des Copolymers in einer Sensoreinheit ermittelt wird, wobei das Copolymer N-isopropyl-acrylamid (NIPAM) und ein Komplexbildner-Monomer (KMX) mit mindestens einer Carbonsäure-Gruppe umfasst und der allgemeinen Formel NIPAMₘ KMXₙ mit m ≥ 1 und n ≥ 1 genügt. Damit kann sowohl ein binäres Copolymer in Betracht kommen, das heißt ein Copolymer, das NIPAM und ausschließlich untereinander gleichartige Komplexbildner-Monomere umfasst, als auch ein Copolymer, das NIPAM und verschiedene Komplexbildner-Monomere umfasst. Als Komplexbildner-Monomere (KMX) können für das Copolymer die der oben genannten Art zum Einsatz kommen.

Die Herstellung einer Sensoreinheit zur Anwendung in einem Sensor zur Ermittlung der Ionenstärke zwei- und/oder höherwertiger Kationen erfolgt, indem zunächst als Sensormaterial zweckmäßigerweise ein Copolymer der allgemeinen Formel NIPAMₘ KMXₙ mit m ≥ 1 und n ≥ 1 vorzugsweise durch radikalische, anionische oder kationische Polymerisation von N-isopropyl-acrylamid (NIPAMₘ) und einem Komplexbildner-Monomer (KMXₙ) mit mindestens einer Carbonsäure-Gruppe synthestisiert wird.

Die mit der Erfindung erzielten Vorteile bestehen insbesondere darin, dass durch die mit dem Medium, z.B. Wasser, in Kontakt bringbare Sensoreinheit, die als Sensormaterial ein Copolymer aufweist, das N-isopropyl-acrylamid (NIPAM) und ein Komplexbildner-Monomer (KMX) mit mindestens einer Carbonsäure-Gruppe umfasst, und das der allgemeinen Formel NIPAMₘ KMXₙ mit m ≥ 1 und n ≥ 1 genügt, ein direkt und kontinuierlich arbeitender Sensor für die Ermittlung der Ionenstärke zwei- und/oder höherwertiger Kationen eingesetzt werden kann. Durch den direkten Einsatz im zu messenden Medium entfällt eine personal- und zeitaufwändige Probenentnahme, so dass eine automatisierte und ständig aktuelle Bestimmung der Ionenstärke möglich ist. Diese ist gerade für den rechtzeitigen Einsatz von Anlagen zur Enthärtung von Ca²⁺- und Mg²⁺-haltigem Wasser von besonderer Bedeutung. Insbesondere durch die Ermittlung eines optischen Transmissionsgrades der mit einem Copolymer als Sensormaterial versehenen Sensoreinheit aufgrund der Phasenseparation des Copolymers infolge Koagulation bei geeigneter Einstellung der Umgebungsparameter in Abhängigkeit von der lonenstärke zwei- und/oder höherwertiger Kationen ist ein unaufwändiges Verfahren zur Ermittlung der entsprechenden Ionenstärke oder Wasserhärte ermöglicht.

Ein Ausführungsbeispiel der Erfindung wird anhand einer Zeichnung näher erläutert. Darin zeigen:
- Figur 1: einen schematischen Aufbau eines Sensors zur Ionenstärke-Bestimmung zwei- und/oder höherwertiger Kationen,
- Figur 2: das Funktionsprinzip des Copolymers in Gegenwart von Ca²⁺- und/ oder Mg²⁺-lonen,
- Figur 3: ein Diagramm des Transmissionsgrades eines ausgewählten Copolymers als Funktion der Wasserhärte,
- Figur 4: ein Diagramm der Phasenseparationstemperatur als Funktion des pH-Wertes am Beispiel von vier ausgewählten Copolymeren,
- Figur 5: ein Diagramm des Transmissionsgrades eines ausgewählten Copolymers als Funktion der Temperatur bei verschiedenen Wasserhärten, und
- Figur 6: ein Diagramm des Transmissionsgrades eines ausgewählten Copolymers als Funktion der Wellenlänge bei verschiedenen Wasserhärten.

Die Bestimmung der Ionenstärke zwei- und/oder höherwertiger Kationen, wie beispielsweise der sogenannten Wasserhärte, ist wichtig bei jedem Betrieb mit strömendem Wasser in allen Bereichen des täglichen Lebens. Die sowohl im Haushalt als auch in der Industrie beispielsweise durch die Wasserhärte hervorgerufenen Ablagerungen führen nämlich nicht nur zu ästhetischen Beeinträchtigungen in Form von Kalkflecken. Mittel- und langfristig führen die Ablagerungen möglicherweise zu gravierenden Schäden an den betroffenen Installationen und Komponenten oder zumindest zu einem höheren Energie-, Reinigungs- oder Waschmittelverbrauch. Somit kann für den gezielten Einsatz von Wasserenthärtungsanlagen die Bestimmung der Ionenstärke der für die Wasserhärte verantwortlichen zweiwertigen Erdalkalimetallionen Ca²⁺ und Mg²⁺ und/oder höherwertigen Kationen notwendig sein.

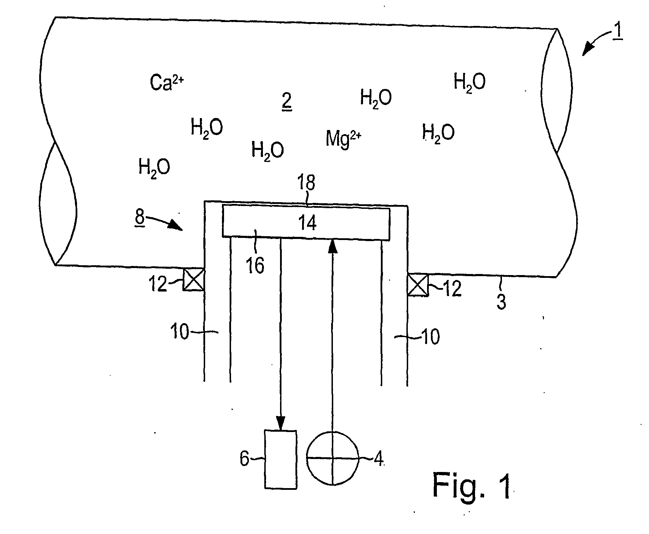

Dazu ist ein Sensor 1 vorgesehen, der in einem eine technische Installation durchströmenden Medium 2, wie beispielsweise Wasser, eingesetzt ist, um dessen Ionenstärke zwei- und/oder höherwertiger Kationen zu bestimmen. Als technische Installation kommt beispielsweise ein Rohr 3 in Betracht. Der in den Figuren 1a,b,c jeweils gezeigte Sensor 1 ist zur Ermittlung der Ionenstärke zwei- und/oder höherwertiger Kationen oder der Wasserhärte auf optischer Basis ausgelegt und somit für einen Einsatz im strömenden oder stehenden Medium 2 unter Verzicht auf Probenahmen besonders geeignet.

Dazu umfasst der jeweilige Sensor 1 je eine Lichtquelle 4 und einen Lichtdetektor 6, die beide nahezu parallel zueinander angeordnet sind, und die im jeweiligen Ausführungsbeispiel beide im gleichen Abstand einer Sensoreinheit 8 zugeordnet sind. Die Sensoreinheit 8 ist dabei ihrerseits am Ende eines in das Rohr 3 hineinragenden Tragrohrs 10 befestigt. Das Tragrohr 10 ist im Ausführungsbeispiel in das Rohr 3 eingeschraubt; alternativ kann aber auch eine andere Verbindung, wie eine Steckverbindung, vorgesehen sein. Zur Verhinderung von einem unerwünschten Medienaustritt ist das Tragrohr 10 über eine umlaufende Dichtung 12 gegenüber dem Rohr 3 abgedichtet. Der Strahlengang der Lichtquelle 4 ist so ausgerichtet, dass das Licht senkrecht auf die in das Rohr 3 gehaltene Sensoreinheit 8 treffen kann. Die Sensoreinheit 8 ist dabei derart ausgelegt, dass sie ihre optischen Eigenschaften, insbesondere ihr Reflexionsvermögen, abhängig von der zu messenden Ionenstärke ändert.

Die Sensoreinheit 8 weist in einer ersten Ausführungsform nach Figur 1a als aktives Sensormaterial eine wässrige Lösung eines Copolymers 14 auf, das in seinem Aufbau derart ausgestaltet ist, dass sich seine optischen Eigenschaften durch die Wechselwirkungen mit den im Medium 2 enthaltenen zwei- und/oder höherwertigen Kationen verändern. Diese Sensoreinheit 8 ist dabei im Ausführungsbeispiel nach Figur 1 a im Inneren eines Messraumes 16 vorgehalten, der von einer für zwei- und/oder höherwertige Kationen permeablen Membran 18 umgeben ist, um durch deren Diffusion durch die Membran 18 einen ausreichenden Kontakt zwischen dem Sensormaterial und dem zu vermessenden Medium 2 herzustellen.

Die optischen Eigenschaften des Copolymers 14 in Abhängigkeit von der Ionenstärke zwei- und/oder höherwertigen Kationen sind allerdings ferner abhängig von spezifischen Parametern der Umgebung, wie pH-Wert, Temperatur und Wellenlänge. So ist für eine geeignete Wahl des pH-Wertes ein Polymer, beispielsweise Polyethylenimid, zur Pufferung des pH-Wertes oder ein Medienüberwachungssystem 22 mit einem pH-Sensor 24 und einer Zuspeiseeinheit 26 für ein Zuspeisemittel 28 vorgesehen, das der Sensoreinheit 8 zugeordnet ist. Der Abhängigkeit von der Temperatur ist über ein Heizelement 30 und einen Temperatur-Sensor 32, mit denen die Sensoreinheit 8 versehen ist, Rechnung getragen. Zur Erzielung einer monochromatischen oder polychromatischen Strahlung mit einer bestimmten Wellenlänge oder einem Wellenlängenintervall ist die Lichtquelle 4 eingesetzt.

Beim Auftreffen des Lichtstrahls auf die Sensoreinheit 8 oder generell auf einen beliebigen Stoff kann das Licht anteilig oder vollständig absorbiert und/oder reflektiert werden. Dabei reduziert sich die Lichtintensität der transmittierten Strahlung, also der durchgelassenen Strahlung, entsprechend der Ausprägung der Absorptions- oder Reflexionseffekte. Der Transmissionsgrad T ist als Quotient aus der durchgelassenen Strahlungsleistung Φ und der auftreffenden Strahlungsleistung Φₒ definiert. Die Größen T, Φ und Φₒ sind aufgrund der Wellenlängenabhängigkeiten von Absorption und Reflexion ebenfalls Funktionen T(λ), Φ(λ) und Φₒ(λ) der Wellenlänge λ. Der Zusammenhang zwischen dem Transmissionsgrad und den stofflichen Eigenschaften des durchstrahlten Stoffes wird durch das Lambert-Beersche Gesetz beschrieben. Danach gilt Ig(1/T(λ)) = Ig(Φₒ(λ)/Φ(λ)) = E(λ) = ε(λ) cd, wobei E(λ) die spektrale Extinktion, ε(λ) der stoffabhängige Extinktionskoeffizient, c die molare Konzentration des absorbierenden oder reflektierenden Stoffes und d die Schichtdicke des durchstrahlten Stoffes angibt.

Aufgrund der Änderung der optischen Eigenschaften des als Sensormaterial eingesetzten Copolymers 14, insbesondere der Abnahme seines Transmissionsgrades oder der Zunahme seiner Reflexion, mit steigender Ionenstärke zwei- und/oder höherwertigen Kationen, ist eine quantitative Bestimmung der Ionenstärke zwei- und/oder höherwertigen Kationen mit optischen Mitteln möglich. Dabei kann der Lichtdetektor 6 des Sensors 1 für die Aufnahme der Transmission oder der Reflexion ausgestaltet sein. Im Ausführungsbeispiel ist der Lichtdetektor 6 beispielsweise als Photodiode ausgestaltet und aus Gründen einer einfacheren Messtechnik für die Aufnahme der Reflexion ausgelegt, da dafür beispielsweise bei einseitiger Anordnung von Lichtquelle 4 und Lichtdetektor 6 kein optischer Spiegel erforderlich ist, der den durchgelassenen Lichtstrahl wieder zum ursprünglichen Strahlengang zurücklenkt.

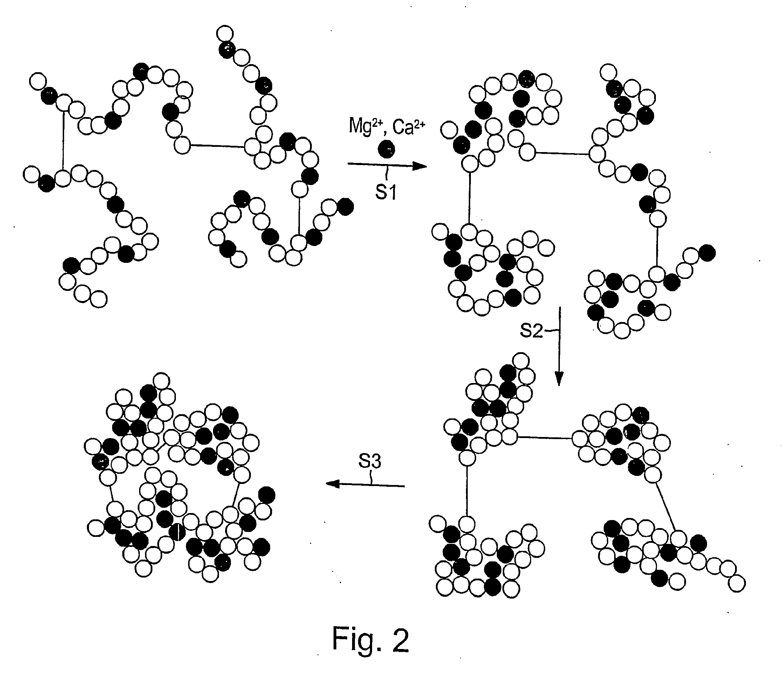

Die optischen Eigenschaften des Copolymers 14 in Abhängigkeit von der Ionenstärke zwei- und/oder höherwertigen Kationen basieren auf dem in Figur 2 dargestellten Funktionsprinzip. In wässriger Lösung liegt der intrinsische pH-Wert eines Copolymers 14 der allgemeinen Formel NIPAMₘ KMXₙ mit m ≥ 1 und n ≥ 1, das durch radikalische, anionische oder kationische Polymerisation von N-isopropyl-acrylamid (NIPAMₘ) und einem Komplexbildner-Monomer (KMXₙ) mit mindestens einer Carbonsäure-Gruppe synthestisiert wird, je nach seiner Konzentration (1 -50 g L⁻¹) zwischen pH ∼ 5 und pH ∼ 3. Durch Zugabe einer definierten Menge Natronlauge als Zuspeisemittel 28 wird die Carbonsäure-Gruppe deprotoniert. In Gegenwart beispielsweise von Magnesium(II)- und/oder Calcium(II)-Kationen, die für die zu bestimmende Ionenstärke oder für die Wasserhärte verantwortlich sind, erfolgt eine Ladungsneutralisation der negativen Ladungen der im Copolymer 14 durch die Deprotonierung gebildeten Carboxylat-Gruppen. Dies führt, wie in Schritt S1 gezeigt, zu einer verringerten Abstoßung und begünstigt eine Assoziation der Polymerstränge. Die individuellen Polymerketten kollabieren in Schritt S2 zu einzelnen Polymerknäueln ("coil to globule transition") und schließen sich anschließend in Schritt S3 bei einer geeigneten Temperatur infolge Koagulation aufgrund ihrer Hydrophobizität zu größeren Clustern zusammen. Denn die Copolymere 14 sind in kaltem Wasser vollkommen löslich, zeigen aber, wie Poly-N-isoprpyl-acrylamid (PNIPAM) auch, von welchem sie abgeleitet sind, das Phänomen der Phasenseparation bei der sogenannten Lower Critical Solution Temperature (LCST). Das heißt, beim Erreichen bzw. oberhalb dieser (kritischen) Temperatur fällt es durch die Bildung hydrophober Domänen aus der Lösung aus. Diese Phasenseparation des Copolymers 14 ist mittels des Lichtdetektors 6 optisch detektierbar, da die Cluster polydispers gebildet werden, somit weiss erscheinen, und daher grundsätzlich das Licht aller Wellenlängen des sichtbaren Spektrums streuen können. Somit wird in Abhängigkeit von der Ionenstärke zwei- und/oder höherwertiger Kationen ein optischer Transmissionsgrad ermittelt, der sich mit der reflektierten Strahlung zu 1 oder 100% ergänzt, die von dem als Photodiode ausgebildeten Lichtdetektor 6 in ein Stromsignal umgewandelt wird.

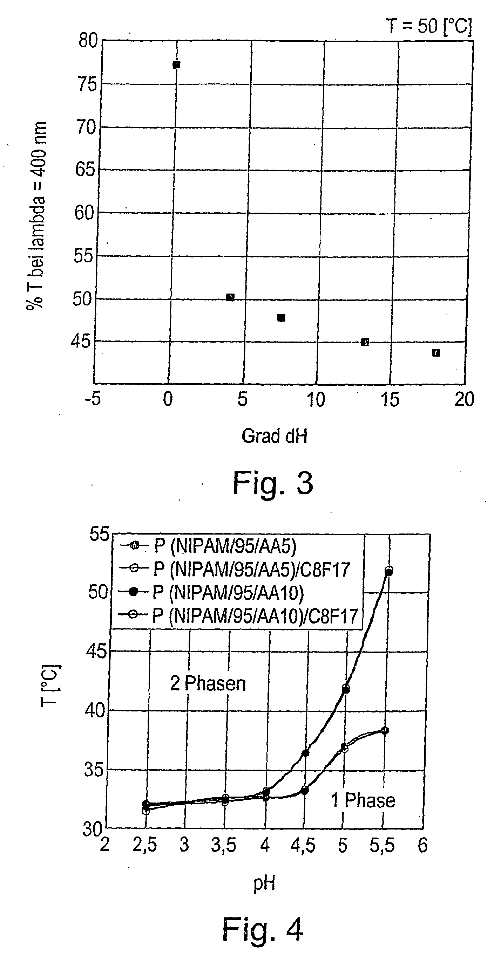

In Figur 3 ist die Abnahme des optischen Transmissionsgrades T in % bei steigender Wasserhärte in °dH am Beispiel eines Copolymers 14 bestehend aus 98% N-isopropyl-acrylamid (NIPAM) und 2% Acrylsäure (AA) bei pH = 8.02, bei einer Temperatur T = 50°C und bei einer durch die Lichtquelle 4 emittierten Wellenlänge λ = 400 nm ersichtlich.

Wie in Figur 4 gezeigt, erfolgt das Ausfallen eines Copolymers 14 am Beispiel von vier Poly(NIPAM/Acrylsäure) mit und ohne organische perfluorierte Seitengruppen aus der Lösung bei niedrigem pH-Wert spontan (ΔT ≈ 3°C). Bei höherem pH-Wert in wässriger Lösung wird generell ein Phasenübergangsbereich durchlaufen (ΔT ≈ 10-15°C), d.h. es ist ein signifikanter Anstieg der LCST-Temperatur und damit der Phasenseparation der Copolymere 14 zu beobachten. In diesem Bereich ist die Konformation des Copolymers 14 im Gegensatz zum Poly-N-isopropyl-acrylamid (PNIPAM) besonders leicht durch Änderungen der Ionenstärke zwei- und/oder höherwertiger Kationen, beispielsweise der Wasserhärte, zu beeinflussen. Die im Copolymer 14 vorhandenen Carbonsäure-Gruppen ermöglichen die Steuerung der LCST als Funktion des pH-Wertes in der wässrigen Lösung. Günstig sind pH = 6.0, pH = 7.0 und pH = 8.0. Dies wird durch Zugabe einer definierten Menge an Natronlauge als Zuspeisemittel 28 erreicht, denn in wässriger Lösung liegt der intrinsische pH-Wert des Copolymers 14 je nach seiner Konzentration (1 - 50 g L⁻¹) zwischen ≈ 5 und ≈ 3. Wird der pH-Wert zu hoch gewählt, unterbleibt je nach dem Anteil organischer Säuren im Copolymer 14 die Phasenseparation des Copolymers 14.

Bei Copolymeren 14, die sich durch ihren unterschiedlichen Anteil an dem Komplexbildner Acrylsäure unterscheiden, trägt der Acrylsäureanteil, der bei pH = 8 nur in seiner Carboxylatform vorliegt, maßgeblich zum Verhalten der Copolymere 14 auf die lonenstärke zwei- und/oder höherwertiger Kationen bei. So ist das Copolymer 14 bestehend aus 98% PNIPAM und 2% Polyacrylsäure (PAA) bereits bei 40°C für den Wasserhärtebreich ab 0° dH geeignet. Das Einsatzgebiet des Copolymers 14 bestehend aus 95% PNIPAM und 5% Polyacrylsäure (PAA) ist hingegen erst bei einer Temperatur von 55°C für den Wasserhärtebereich ab ∼ 4° dH und für das Copolymer 14 bestehend aus 90% PNIPAM und 10% Polyacrylsäure (PAA) erst bei einer Temperatur von 70°C für den Wasserhärtebereich ab ∼ 7,5° dH geeignet. Höhere Wasserhärtebereiche bedürfen einer geringeren Temperatur, so dass für das Copolymer 14 bestehend aus 95% PNIPAM und 5% Polyacrylsäure (PAA) bei 45°C und für das Copolymer 14 bestehend aus 90% PNIPAM und 10% Polyacrylsäure (PAA) bei 60° C ein Wasserhärtebereich ab 18° dH geeignet ist.

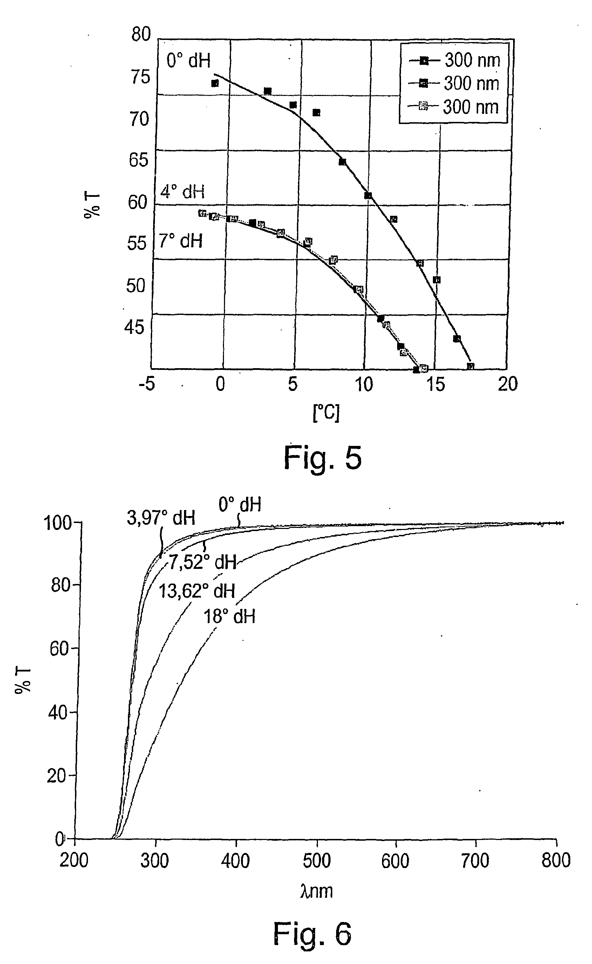

In Figur 5 sind die Transmissionsgrade T in % eines Copolymers 14, bestehend aus 80% PNIPAM und 20% Polyacrylsäure (PAA) als Funktion der Temperatur T in °C bei pH = 8.02 und bei einer Wellenlänge λ = 300 nm dargestellt. In der Figur 5 ist im Bereich um 45 °C ein signifikanter Unterschied, nämlich eine signifikante Absenkung, des Transmissionsgrades T beim Anstieg des Deutschen Härtegrades dH von 0° auf 4° ersichtlich. Bei höheren Temperaturen werden die hydrophoben Wechselwirkungen stärker, die die elektrostatischen Abstoßungen kompensieren, so dass die Unterschiede der Transmissionsgrade T beim Anstieg des Deutschen Härtegrades dH von 0° auf 4° wieder geringer werden. Daher wird bei einer Temperatur T= 45°C eine optimale Empfindlichkeit des Copolymers 14 bestehend aus 80% PNIPAM und 20% Poiyacrylsäure (PAA) gefunden.

In Figur 6 sind die Transmissionsgrade T in % eines Copolymers 14, bestehend aus 98% PNIPAM und 2% Polyacrylsäure (PAA) bei pH = 8 und einer konstanten Temperatur T = 45°C als Funktion der Wellenlänge λ von 200 bis 800 nm bei verschiedenen Wasserhärten dH dargestellt. Bei λ = 300 nm und 400 nm ist im Gegensatz zu größeren Wellenlängen eine signifikante Absenkung des Transmissionsgrades T beim Anstieg des Deutschen Härtegrade dH zwischen 0° und 18° ersichtlich. Daher ist für einen technisch relevanten Einsatzbereich mit einem geringen Kosten- und Herstellungsaufwand eine Bestrahlung mit einer Wellenlänge λ von 300 nm oder 400 nm eingestellt.

Die Synthese von Poly-N-isopropyl-acrylamid/ Methacrylsäure-Copolymeren ist kann beispielsweise nach dem in Journal of Physical Chemistry B, 1998, 102(8), 1364-1371 beschriebenen Verfahren wie folgt vorgenommen werden. In einem 250 ml Glasreaktor werden 1,5 g einer Mischung aus Methacrylsäure-Monomer (MMA) und N-isopropyl-acrylamid-Monomer (NIPAM) mit unterschiedlichen molaren Verhältnissen zusammen mit 1,0 mol% Methylenbisacrylamid (BIS) (des Gesamtmonomers) sowie 0,04 g Natriumdodecylsulfat (SDS) in 100 ml deionisiertem und filtriertem Wasser gemischt. Die Mischung wird unter Stickstoffatmosphäre und Rückflusskühlung in einem Wasserbad auf 70°C erhitzt und 40 Minuten lang mit einem Glasrührstab mit einem 6 cm Teflon-Paddel bei 300 Umdrehungen pro Minute gerührt. Dann werden 0,065 g Ammoniumpersulfat (APS) in 0,3 ml Wasser gelöst und zum Start der Reaktion hinzugefügt. Die Reaktionsmischung wird abhängig vom Ausgangsmaterial zwischen ½ und 4 Stunden gerührt. Die polymerisierte Matrix wird durch Zentrifugieren, Dekantieren und wiederholtes Dispergieren in deionisiertem Wasser (pH ∼ 5,6) gereinigt, um SDS und andere Verunreinigungen zu entfernen und abschließend für ein konstantes Gewicht bei 45°C vakuumgetrocknet.

Zur Bildung eines Makromoleküls mit einer Molekularmasse größer 2.000.000 für eine ausreichende chemische Langzeitstabilität erfolgt die chemische Vernetzung der individuellen Polymerstränge des Copolymers 14 im Ausführungsbeispiel nach der Synthese des eigentlichen Copolymers 14 unter Knüpfung einer Carbonsäureamidbindung über Amin-terminierte Polyoxyethylen-Brücken, Amin-terminierte Polyoxyethylen-Brücken-substituierte Aromaten, Amin-terminierte Polyimine, Amin-terminierte aliphatische Kohlenwasserstoffe, Amin-terminierte aliphatisch-aromatische Kohlenwasserstoffe mit beliebigem Substitutionsmuster oder eine Kombination der vorgenannten Strukturelemente. Dabei kann die Verknüpfung beispielsweise durch ein wasserentziehendes Reagenz (z.B. Dicyclohexyl-carbodiimid, DCC) und unter Verwendung eines Katalysators, welcher einen intermediären Aktivester bildet (z.B. N-Hydroxy-succinimid, NHS), vorgenommen werden. Hierbei ist die Konzentration des Vernetzers gering zu halten (0.1 - 2.0 Gewichtsprozente der Gesamtmasse der synthetisierten Copolymere 14). Als Lösungsmittel kommen Tetrahydrofuran, Dioxan, Dimethylformamid, Dimethylacetamid oder Chloroform in Frage. Die Umfällung des durch die Vernetzung des Copolymers 14 gebildeten Makromoleküls kann durch Dispergieren in Tetrahydrofuran und Eingießen in einen Überschuss eines n-Alkans (oder einer Mischung aus verschiedenen Alkanen) oder durch Lösung in Wasser und Eingießen in einen Alkohol (C1-C4), Tetrahydrofuran oder Dimethylformamid bzw. Dimethylacetamid erfolgen. Die Wiedergewinnung der umgefällten Makromoleküle erfolgt durch Filtration.

### Bezugszeichenliste

- 1: Sensor
- 2: Medium
- 3: Rohr
- 4: Lichtquelle
- 6: Lichtdetektor
- 8: Sensoreinheit
- 10: Tragrohr
- 12: Dichtung
- 14: Copolymer
- 16: Messraum
- 18: Membran
- 20: Träger
- 22: Medienüberwachungssystem
- 24: pH-Sensor
- 26: Zuspeiseeinheit
- 28: Zuspeisemittel
- 30: Heizelement
- 32: Temperatur-Sensor
- S1, S2, S3: Schritt

## Patentansprüche

1. Sensor (1) zur Ermittlung der Ionenstärke zwei- und/oder höherwertiger Kationen in einem Medium (2) mit einer mit dem Medium (2) in Kontakt bringbaren Sensoreinheit (8), die als Sensormaterial ein Copolymer (14) aufweist, das N-isopropyl-acrylamid (NIPAM) und ein Komplexbildner-Monomer (KMX) mit mindestens einer Carbonsäure-Gruppe umfasst, und das der allgemeinen Formel NIPAMₘ KMXₙ mit m ≥ 1 und n ≥ 1 genügt, wobei das Copolymer (14) in der Sensoreinheit (8) in einem von einer Membran umgebenen Messraum in wässriger Lösung mit einem pH-Wert von etwa 6 bis 8 vorgehalten ist.

2. Sensor (1) nach Anspruch 1, dessen Sensoreinheit (8) zur Einstellung des pH-Werts eine Zuspeiseeinheit (26) für ein Zuspeisemittel (28) zugeordnet ist.

3. Sensor (1) nach einem der Ansprüche 1 bis 3, bei dem NIPAMₘ eine Massenkonzentration im Copolymer (14) größer 50% aufweist.

4. Sensor (1) nach Anspruch 1 oder 2, bei dem das Komplexbildner-Monomer (KMX) mit zwei- und/oder höherwertigen Kationen eine freie Sindungsenthalpie kleiner als ΔG = - 35 KJ/mol aufweist.

5. Sensor (1) nach einem der vorhergehenden Ansprüche, bei dem als Komplexbildner-Monomer (KMX) für das Copolymer (14) Acrylsäure, {[4-Acryloyl-amino-2-(bis-carboxy-methyl-amino)-phenyl]-carboxy-methyl-amino}-essigsäure, {[4-Acryloyl-amino-2-(bis-carboxy-methyl-amino)-cyclohexyl]-carboxy-methyl-amino}-essigsäure, ({2-[Acryloyl-(2-dicarboxy-methylamino-ethyl)-amino]-ethyl}-carboxymethyl-amino)-essigsäure, [({Acryloyl-[(dicarboxymethyl-amino)-methyl]-amino}-methyl)-carboxymethyl-amino]-essigsäure, Methacrylsäure, organisches Acrylat, organisches Methacrylat, Zimtsäure oder ein Derivat der Zimtsäure eingesetzt ist.

6. Sensor (1) nach einem der vorhergehenden Ansprüche, bei dem das Copolymer (14) eine mittlere Molekularmasse im Bereich zischen 40.000 und 200.000 aufweist.

7. Sensor (1) nach einem der vorhergehenden Ansprüche, bei dem das Copolymer (14) hinsichtlich seines Gehalts an Carbonsäuro-Gruppen und/ oder deren Art an einen vorgegebenen Messbereich für die Ionenstärke der zwei- und/oder höherwertigen Kationen angepasst ist.

8. Sensor (1) nach einem der vorhergehenden Ansprüche, bei dem individuelle Polymerstränge des Copolymers (14) zur Bildung eines Makromoleküls vernetzt sind, wobei das Makromolekül eine Molekularmasse größer 2.000.000 aufweist.

9. Sensor (1) nach Anspruch 8, bei dem die individuellen Polymerstränge des Copolymers (14) über Divinylbenzol oder substituierte Butadiene vernetzt sind.

10. Sensor (1) nach Anspruch 8, bei dem die individuellen Polymerstränge des Copolymers (14) über Amin-terminierte Polyoxyethylen-Brücken, Amin-terminierte Polyoxyethylen-Brücken-substituierte Aromaten, Amin-terminierte Polyimine, Amin-terminierte aliphatische Kohlenwasserstoffe und/oder Amin-terminierte aliphatisch-aromatische Kohlenwasserstoffe vernetzt sind.

11. Sensor (1) nach einem der vorhergehenden Ansprüche, wobei die Membran (18) für zwei- und/oder höherwertige Kationen permeabel ist.

12. Sensor (1) nach einem der vorhergehenden Ansprüche, bei dem der Sensoreinheit (8) zur Auswertung der optischen Eigenschaften des Copolymers (14) eine Lichtquelle (4) und ein Lichtdetektor (6) zugeordnet sind.

13. Sensor (1) nach Anspruch 14, bei dem die Lichtquelle (4) mit einer Wellenlänge oder einem Weitentangenintervall im Bereich von 300 nm bis 500 nm ausgestaltet ist.

14. Sensor (1) nach einem der vorhergehenden Ansprüche, bei dem das Copolymer (14) von einem Polymer umgeben ist, das den pH-Wert der wässrigen Lösung des Copolymers (14) puffert.

15. Sensor (1) nach einem der vorhergehenden Ansprüche, bei dem der Sensoreinheit (8) ein Medienüberwachungssystem (22) mit einem pH-Sensor (24) und einer Zuspeiseeinheit (26) für ein Zuspeisemittel (28) zugeordnet ist.

16. Sensor (1) nach einem der vorhergehenden Ansprüche, bei dem die Sensoreinheit (8) mit einem Heizelement (30) und einem Temperatur-Sensor (32) versehen ist.

17. Verfahren zur Ermittlung der Ionenstärke zwei- und/oder höherwertiger Kationen, bei dem ein optischer Transmissionsgrad einer mit einem Copolymer (14) als Sensormaterial versehenen Sensoreinheit (8) ermittelt wird, wobei das Copolymer (14) N-isopmpyl-acrylamid (NIPAM) und ein Komplexbildner-Monomer (KMX) mit mindestens einer Carbonsäure-Gruppe umfasst und der allgemeinen Formel NIPAMₘ KMXₙ mit m ≥ 1 und n ≥ 1 genügt, und wobei das Copolymer (14) in einem von einer Membran umgebenen Messraum in wässriger Lösung vorgehalten wird, deren pH-Wert auf etwa 6 bis 8 eingestellt wird.

18. Verwendung eines Sensor (1) nach einem der Ansprüche 1 bis 18 zur Ermittlung der Ionenstärke zwei- und/oder hoherwertiger Kationen.

19. Verwendung eines Sensors (1) nach einem der Ansprüche 1 bis 18 als Wasserhärte-Sensor.

## Claims

1. Sensor (1) for determining the ionic strength of bivalent and/or higher-valent cations in a medium (2), having a sensor unit (8) which can be brought into contact with the medium (2) and which includes as sensor material a copolymer (14) comprising N-isopropylacrylamide (NIPAM) and a complexing monomer (KMX) with at least one carbonic-acid group and fitting the general formula NIPAMₘKMXₙ, with m ≥ 1 and n ≥ 1, the copolymer (14) being provided in the sensor unit (8) in a measuring room surrounded by a diaphragm, in aqueous solution with a pH value of approximately 6 to 8.

2. Sensor (1) according to claim 1, wherein a feeding unit (26) for a feeding means (28) is associated to the sensor unit (8) for adjusting the pH value.

3. Sensor (1) according to any of claims 1 to 3, wherein NIPAMₘ has a mass concentration in the copolymer (14) higher than 50 %.

4. Sensor (1) according to claim 1 or 2, wherein the complexing monomer (KMX) has a free bonding enthalpy with bivalent and/or higher-valent cations of less than ΔG = -35 kJ/mol.

5. Sensor (1) according to any of the preceding claims, wherein acrylic acid, ([4-acryloyl-amino-2-(bis-carboxy-methyl-amino)-phenyl]-carboxy-methyl-amino) ethanoic acid, ([4-acryloyl-amino-2-(bis-carboxy-methyl-amino)-cyclohexyl]-carboxy-methyl-amino) ethanoic acid, ((2-[acryloyl-(2-dicarboxy-methyl-aminoethyl)-amino]-ethyl)-carboxy-methyl-amino) ethanoic acid, [((acryloyl-[(dicarboxymethyl-amino)-methyl]-amino)-methyl)-carboxy-methyl-amino] ethanoic acid, methacrylic acid, organic acrylate, organic methacrylate, cinnamic acid or a derivative of cinnamic acid is used as complexing monomer (KMX) for the copolymer (14).

6. Sensor (1) according to any of the preceding claims, wherein the copolymer (14) has a mean molecular mass in the range between 40,000 and 200,000.

7. Sensor (1) according to any of the preceding claims, wherein the copolymer (14), as to its content of carbonic-acid groups and/or as to the latter's type is adapted to a preset measuring range for the ionic strength of the bivalent and/or higher-valent cations.

8. Sensor (1) according to any of the preceding claims, wherein the individual polymer strings of the copolymer (14) are cross-linked to form a macromolecule, the macromolecule having a molecular mass larger than 2,000,000.

9. Sensor (1) according to claim 8, wherein the individual polymer strings of the copolymer (14) are cross-linked via divinylbenzene or substituted butadienes.

10. Sensor (1) according to claim 8, wherein the individual polymer strings of the copolymer (14) are cross-linked via amin-terminated polyoxyethylene bridges, aromatics substituted by amin-terminated polyoxyethylene bridges, amin-terminated polyimines, amin-terminated aliphatic hydrocarbons and/or amin-terminated aliphatic-aromatic hydrocarbons.

11. Sensor (1) according to any of the preceding claims, wherein the diaphragm (18) is permeable for bivalent and/or higher-valent cations.

12. Sensor (1) according to any of the preceding claims, wherein a light source (4) and a light detector (6) are associated to the sensor unit (8) for evaluating the optical properties of the copolymer (14).

13. Sensor (1) according to claim 14, wherein the light source (4) is designed with a wave length or a wave-length interval in the range between 300 nm and 500 nm.

14. Sensor (1) according to any of the preceding claims, wherein the copolymer (14) is surrounded by a polymer buffering the pH value of the aqueous solution of the copolymer (14).

15. Sensor (1) according to any of the preceding claims, wherein a medium-monitoring system (22) with a pH sensor (24) and a feeding unit (28) for a feeding means (28) is associated to the sensor unit (8).

16. Sensor (1) according to any of the preceding claims, wherein the sensor unit (8) is provided with a heating element (30) and a temperature sensor (32).

17. Method for determining the ionic strength of bivalent and/or higher-valent cations, wherein an optical transmission degree of a sensor unit (8) provided with a copolymer (14) as sensor material is determined, the copolymer (14) comprising N-isopropylacrylamide (NIPAM) and a complexing monomer (KMX) with at least one carbonic-acid group and fitting the general formula NIPAMₘKMXₙ, with m ≥ 1 and n ≥ 1, the copolymer (14) being provided in the sensor unit (8) in a measuring room surrounded by a diaphragm, in aqueous solution with a pH value of approximately 6 to 8.

18. Use of a sensor (1) according to any of claims 1 to 18 for determining the ionic strength of bivalent and/or higher-valent cations.

19. Use of a sensor (1) according to any of claims 1 to 18 as a water-hardness sensor.

## Revendications

1. Détecteur (1) pour déterminer la force ionique de cations bivalents et/ou à valence plus élevée dans un milieu (2), ayant un bloc détecteur (8) qui peut être mis en contact avec le milieu (2) et qui inclut en tant que matériau de détecteur un copolymère (14) comprenant du N-isopropylacrylamide (NIPAM) et un monomère complexant (KMX) avec au moins un groupe de dioxyde de carbone et satisfaisant la formule générale NIPAMₘKMXₙ, avec m ≥ 1 et n ≥ 1, le copolymère (14) étant contenu dans le bloc détecteur (8) dans un espace de mesurage entouré par un diaphragme, dans une solution aqueuse avec une valeur pH d'environ 6 à 8.

2. Détecteur (1) selon la revendication 1, dans lequel une unité d'alimentation (26) pour un moyen d'alimentation (28) est affectée au bloc détecteur (8) pour régler la valeur pH.

3. Détecteur (1) selon l'une quelconque des revendications 1 à 3, dans lequel NIPAMₘ a une concentration massique dans le copolymère (14) de plus de 50 %.

4. Détecteur (1) selon la revendication 1 ou 2, dans lequel le monomère complexant (KMX) a une enthalpie libre de liaison avec les cations bivalents et/ou à valence plus élevée de moins de ΔG = -35 kJ/mol.

5. Détecteur (1) selon l'une quelconque des revendications précédentes, dans lequel de l'acide acrylique, de l'acide acétique ([4-acryloyl-amino-2-(bis-carboxy-méthyl-amino)-phényl]-carboxy-méthyl-amino), de l'acide acétique ([4-acryloyl-amino-2-(bis-carboxy-méthyl-amino)-cyclohexyl]-carboxy-méthyl-amino), de l'acide acétique ((2-[acryloyl-(2-dicarboxy-méthyl-amino-éthyl)-amino]-éthyl)-carboxy-méthyl-amino), de l'acide acétique [((acryloyl-[(dicarboxy-mèthyl-amino)-méthyl]-amino)-méthyl)-carboxy-méthyl-amino], de l'acide méthacrylique, de l'acrylate organique, du méthacrylate organique, de l'acide cinnamique ou un dérivé de l'acide cinnamique est utilisé comme monomère complexant (KMX) pour le copolymère (14).

6. Détecteur (1) selon l'une quelconque des revendications précédentes, dans lequel le copolymère (14) a une masse moléculaire moyenne dans le domaine entre 40.000 et 200.000.

7. Détecteur (1) selon l'une quelconque des revendications précédentes, dans lequel le copolymère (14), en ce qui concerne son teneur en groupes de dioxyde de carbone et/ou en ce qui concerne le type de ces dernières, est adapté à un champ de mesure prédéfini pour la force ionique des cations bivalents et/ou à valence plus élevée.

8. Détecteur (1) selon l'une quelconque des revendications précédentes, dans lequel les faisceaux individuels de polymère, du copolymère (14), sont réticulés pour former un macromolécule, ce macromolécule ayant une masse moléculaire de plus de 2.000.000.

9. Détecteur (1) selon la revendication 8, dans lequel les faisceaux individuels de polymère, du copolymère (14), sont réticulés par divinylbenzène ou par des butadiènes substitutées.

10. Détecteur (1) selon la revendication 8, dans lequel les faisceaux individuels de polymère, du copolymère (14), sont réticulés par des ponts de polyoxyéthylène terminés par une amine, des aromatiques substitués par des ponts de polyoxyéthylène terminés par une amine, des polyimines terminés par une amine, des hydrocarbures aliphatiques et/ou des hydrocarbures aliphatiques-aromatiques terminés par une amine.

11. Détecteur (1) selon l'une quelconque des revendications précédentes, dans lequel le diaphragme (18) est perméable aux cations bivalents et/ou à valence plus élevée.

12. Détecteur (1) selon l'une quelconque des revendications précédentes, dans lequel une source lumineuse (4) et un capteur de lumière (6) sont affectée au bloc détecteur (8) pour évaluer les propriétés optiques du copolymère (14).

13. Détecteur (1) selon la revendication 14, dans lequel la source lumineuse (4) est conçue avec une longueur d'onde ou un intervalle de longueur d'onde dans le domaine entre 300 nm et 500 nm.

14. Détecteur (1) selon l'une quelconque des revendications précédentes, dans lequel le copolymère (14) est entouré d'un polymère tamponnant la valeur pH de la solution aqueuse du copolymère (14).

15. Détecteur (1) selon l'une quelconque des revendications précédentes, dans lequel un système de monitorage du milieu (22) avec un détecteur pH (24) et une unité d'alimentation (28) pour un moyen d'alimentation (28) est affectée au bloc détecteur (8).

16. Détecteur (1) selon l'une quelconque des revendications précédentes, dans lequel le bloc détecteur (8) est muni d'un élément de chauffage (30) et d'un palpeur de température (32).

17. Méthode pour déterminer la force ionique de cations bivalents et/ou à valence plus élevée, dans lequel un degré de transmission optique d'un bloc détecteur (8) muni d'un copolymère (14) en tant que matériau de détecteur est déterminé, le copolymère (14) comprenant du N-isopropylacrylamide (NIPAM) et un monomère complexant (KMX) avec au moins un groupe de dioxyde de carbone et satisfaisant la formule générale NIPAMₘKMXₙ, avec m ≥ 1 et n ≥ 1, le copolymère (14) étant contenu dans le bloc détecteur (8) dans un espace de mesurage entouré par un diaphragme, dans une solution aqueuse avec une valeur pH d'environ 6 à 8.

18. Utilisation d'un détecteur (1) selon l'une quelconque des revendications 1 à 18 pour déterminer la force ionique de cations bivalents et/ou à valence plus élevée.

19. Utilisation d'un détecteur (1) selon l'une quelconque des revendications 1 à 18 en tant que détecteur de la dureté de l'eau.
